# EUROPÄISCHE PATENTSCHRIFT

(11) **EP 1 316 295 B1**
(45) Veröffentlichungstag und Bekanntmachung des Hinweises auf die Patenterteilung: **12.10.2005**
(21) Anmeldenummer: 02025337.3
(22) Anmeldetag: 13.11.2002
(51) Int. Cl.: A61B 17/70

(54) **Element mit einem Schaft und einem damit verbundenen Halteelement zum Verbinden mit einem Stab**
Element with a shaft and a retaining element connected to the shaft for attaching a rod
Elément avec une vis d'ancrage et un élément de retenue relié à la vis pour connecter une barre

(30) Priorität: 27.11.2001 DE 10157969
(43) Veröffentlichungstag der Anmeldung: 04.06.2003
(73) Patentinhaber: BIEDERMANN MOTECH GmbH, 78054 VS-Schwenningen (DE)
(72) Erfinder: Biedermann, Lutz, 78048 VS-Villingen (DE); Harms, Jürgen, 76227 Karlsruhe (DE)
(74) Vertreter: Prüfer, Lutz H.

(56) Entgegenhaltungen:
- WO-A-95/01132
- DE-U- 29 810 798
- SU-A- 371 359
- US-A- 2 783 809
- US-A- 4 850 775
- US-A- 5 536 268
- US-A- 6 063 090
- US-A- 6 074 391
- PATENT ABSTRACTS OF JAPAN Bd. 1996, Nr. 09, 30. September 1996 (1996-09-30) & JP 8 112291 A (TERUMO CORP), 7. Mai 1996 (1996-05-07)

## Beschreibung

Die Erfindung betrifft ein in der Wirbelsäulen- bzw. Unfallchirurgie zu verwendendes Element mit einem Schaft und einem damit verbundenen Halteelement zum Verbinden mit einem Stab.

Aus der EP 0 614 649 ist eine Knochenschraube mit einem Schaft und einem damit verbundenen Halteelement zum Verbinden mit einem Stab nach dem Oberbegriff des Patentanspruches 1 bekannt. Bei dieser beschriebenen sogenannten Polyaxial-Knochenschraube zeigt das Innengewinde der freien Schenkel des Halteelements bzw. Aufnahmeteils und das Außengewinde der Innenschraube im wesentlichen ein Rundgewinde.

Aus der US 5,005,562 und der US 6,074,391 sind jeweils ein Element nach dem Oberbegriff des Patentanspruches 1 bekannt. Das Innengewinde der freien Schenkel des Halteelements bzw. Aufnahmeteils und das Außengewinde der Innenschraube sind mit einer Sägezahnstufe zum Vermeiden des Aufspreizens der Schenkel durch radial wirkende Kräfte beim Einschrauben ausgebildet.

Aus der WO 00/27 297 ist ebenfalls ein Element nach dem Oberbegriff des Patentanspruches 1 bekannt. Bei diesem Element schließen die Flanken des Innengewindes, die den freien Enden der durch die U-förmige Ausnehmung gebildeten Schenkel abgewandt sind, mit einer zu der zentralen Achse des Aufnahmeteils senkrechten Ebene einen negativen Winkel ein. Dadurch soll ebenfalls das Aufspreizen der Schenkel verhindert werden.

Bei den oben beschriebenen Vorrichtungen ist entweder eine das Halteelement von außen umfassende Vorrichtung zum Verhindern des Aufspreizens der Schenkel erforderlich oder das zu verwendende Gewinde ist schwierig herzustellen. Dies trifft insbesondere für das aus der WO 00/27 297 bekannte Gewinde mit dem negativen Flankenwinkel zu.

Aus der DE 298 10 798 U1 ist eine Knochenschraube mit einem eine Nut aufweisenden Gabelkopf zum Verbunden mit einem Stab und mit einer den Stab fixierenden Machenschraube bekannt. Das Innengewinde des Gabelkopfs weist eine ebene obere Flanke und eine stufenartig ausgebildete untere Flanke auf, wobei die Stufe der unteren Flanke derart geformt, ist daß sie einen Hinterschnitt bildet.

Es ist Aufgabe der Erfindung, ein Element mit einem Schaft und einem damit verbundenen Halteelement zum Verbinden mit einem Stab bereitzustellen, welches einfach herzustellen, kompakt in der Bauweise und leicht zu bandhaben ist.

Die Aufgabe wird gelöst durch ein Element gemäß Patentanspruch 1. Weiterbildungen der Erfindung sind in den Unteransprüchen angegeben.

Das erfindungsgemäße Element weist den Vorteil auf, dass das Innengewinde der Schenkel bzw. das Außengewinde des Verschlusselements, welche als Flachgewinde ausgebildet sind, äußerst einfach herzustellen ist. Dadurch werden Produktionskosten gespart und die Präzision kann erhöht werden. Da mit diesem Gewinde keine Kräfte in radialer Richtung nach außen wirken, wird ein Aufspreizen der freien Schenkel des Halteelements vermieden und damit ist kein zusätzliches von außen anzubringendes Element zum Vermeiden des Aufspreizens erforderlich. Dadurch wird eine kompaktere Bauweise ermöglicht. Weitere Merkmale und Zweckmäßigkeiten der Erfindung ergeben sich aus der Beschreibung von Ausführungsbeispielen anhand der Figuren.

Von den Figuren zeigen:
- Fig. 1: eine Schnittdarstellung einer ersten Ausführungsform des erfindungsgemäßen Elements;
- Fig. 2a: eine Teilansicht der Ausführungsform von von Fig. 1 mit noch nicht festgezogener Innenschraube;
- Fig. 2b: eine vergrößerte Darstellung eines Details von Fig. 2a;
- Fig. 3a: eine Teilansicht der Ausführungsform von von Fig. 1 mit festgezogener Innenschraube;
- Fig. 3b: eine vergrößerte Darstellung eines Details von Fig. 3a;
- Fig. 4: eine Schnittdarstellung einer zweiten Ausführungsform;
- Fig. 5: eine Schnittdarstellung einer dritten Ausführungsform;
- Fig. 6: eine perspektivische Ansicht der Mutter von Fig. 5;
- Fig. 7: eine Teilansicht im Schnitt einer Abwandlung der dritten Ausführungsform; und
- Fig. 8: eine perspektivische Ansicht der Mutter der Ausführungsform von Fig. 7.

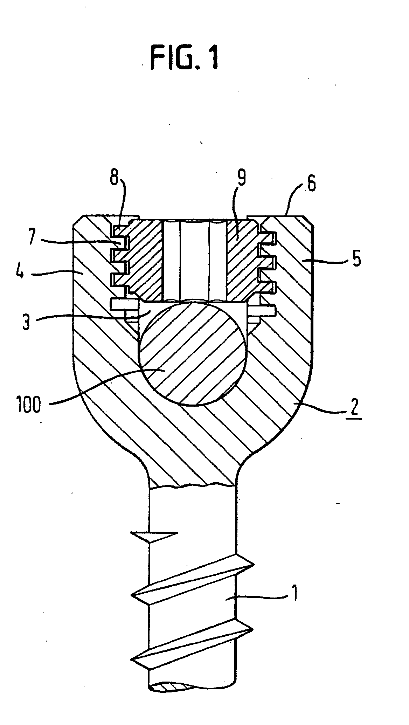

Das erfindungsgemäße Element ist in der in der in den Figuren Fig. 1 dargestellten Ausführungsform als Monoaxial-Knochenschraube ausgebildet. Diese weist einen Schaft 1 mit einem Knochengewindeabschnitt und ein damit starr verbundenes Aufnahmeteil 2 zur Aufnahme eines die Knochenschraube mit weiteren Knochenschrauben verbindenden Stabes 100 auf. Hierzu ist das Aufnahmeteil mit einer Ausnehmung 3 mit einem U-förmigen Querschnitt versehen, die gerade so groß bemessen ist, daß der Stab 100 einlegbar ist und in den Grund der Ausnehmung passt. Durch die U-förmige Ausnehmung 3 sind zwei freie Schenkel 4, 5 mit jeweils einem freien Ende 6 gebildet, welches den oberen Rand des Aufnahmeteils 2 bildet. Angrenzend an das freie Ende 6 weisen die Schenkel 4, 5 ein Innengewinde 7 auf, welches mit einem entsprechenden Außengewinde 8 einer zwischen die Schenkel 4, 5 einzuschraubenden Innenschraube 9 zum Fixieren des Stabs 100 zusammenwirkt.

Wie insbesondere in den Figuren 1 bis 3b ersichtlich ist, ist das Innengewinde 7 und entsprechend dazu das Außengewinde 8 als Flachgewinde ausgebildet. Dieses ist dadurch gekennzeichnet, dass die Gewindeflanken 7a, 7b des Innengewindes mit der Mittenachse M des Aufnahmeteils jeweils einen Winkel von 90° einschließen. Entsprechend schließen die Gewindeflanken 8a, 8b des Außengewindes der Innenschraube 9 mit der Schraubenachse S einen Winkel von 90 ° ein. Der Gewindequerschnitt ist im wesentlichen rechteckig. Ferner können die Kanten abgerundet ausgebildet sein. Die Gewindeflanken sind ohne Hinterschneidungen ausgebildet.

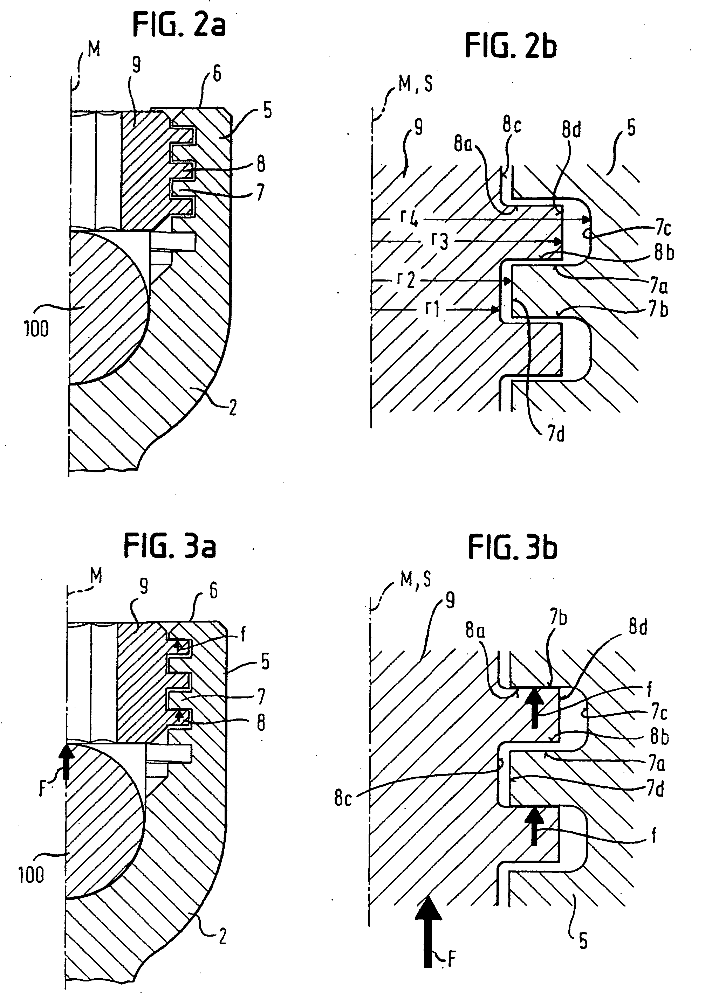

Wie sich insbesondere aus der vergrößerten Darstellung gemäß Fig. 2b und Fig. 3b ergibt, sind bei diesem Ausführungsbeispiel das Innengewinde 7 der Schenkel und das Außengewinde 8 der Innenschraube 9 wie folgt relativ zueinander bemessen: Der Radius r₁ von der Schraubenachse S bis zum Gewindegrund 8c der Innenschraube 9 ist kleiner als der Radius r₂ von der Mittenachse M des Aufnahmeteils 2 bis zu seiner Wendelspitze 7d. Der Radius r₃ von der Schraubenachse S bis zur Wendelspitze 8d der Innenschraube 9 ist kleiner als der Radius r₄ von der Mittenachse M des Aufnahmeteils 2 bis zum Gewindegrund 7c seines Innengewindes.

Ferner sind, wie in Fig. 2b gezeigt ist, die Abmessungen der Gewinde derart, dass beim unbelasteten Ineingriffbringen von Innenschraube und freien Schenkeln 4, 5 des Aufnahmeteils ein Unterschied der Radien r₁ und r₂, d.h. ein radiales Spiel, von 1% bis 5% des Gewindeaußendurchmessers, bevorzugt etwa 1% besteht. In axialer Richtung besteht bei einem Einstich von etwa 5mm ein axiales Spiel von etwa 10%.

Im Betrieb wird nach Einlegen des Stabes in das Aufnahmeteil 2 die Innenschraube 9 zunächst locker eingeschraubt, so daß der Stab noch justiert werden kann. Zum Fixieren des Stabes 100 wird die Innenschraube 9 festgezogen. Dadurch erfährt sie eine in den Figuren 3a und 3b durch den Pfeil F dargestellte Gegenkraft. Im Ergebnis gelangen die dem freien Ende 6 zugewandten Flanken 8a des Außengewindes und die dem freien Ende abgewandten Flanken 7b des Innengewindes aufeinander. Die auf die Gewindeflanken wirkende Kraft wirkt dabei nur in axialer Richtung, wie durch die kurzen Pfeile f in den Figuren 3a und 3b dargestellt ist. Daher erfolgt kein Aufspreizen der Schenkel 4, 5.

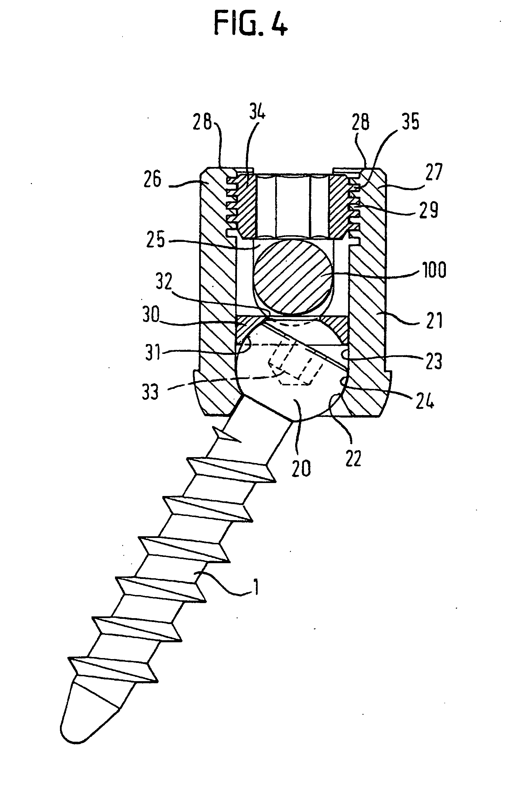

In einer zweiten, in Fig. 4 gezeigten Ausführungsform ist das erfindungsgemäße Element als Polyaxial-Knochenschraube ausgebildet. Die Polyaxial-Knochenschraube weist ein Schraubenelement auf mit einem Gewindeschaft 1 mit einem Knochengewinde, der über einen kugelsegmentförmigen Kopf 20 mit einem Aufnahmeteil 21 verbunden ist. Das Aufnahmeteil 21 hat an seinem einen Ende eine axialsymmetrisch ausgerichtete erste Bohrung 22, deren Durchmesser größer als der des Gewindeabschnitts des Schafts 1 und kleiner als der des Kopfs 20 ist. Ferner weist das Aufnahmeteil 21 eine koaxiale zweite Bohrung 23 auf, die auf dem der ersten Bohrung 22 gegenüberliegenden Ende offen ist und deren Durchmesser so groß ist, daß das Schraubenelement durch das offene Ende mit seinem Gewindeabschnitt durch die erste Bohrung 22 hindurch und mit dem Kopf 20 bis zum Grund der zweiten Bohrung 23 führbar ist. Zwischen der ersten und der zweiten Bohrung ist ein kleiner koaxialer Abschnitt 24 vorgesehen, der unmittelbar an die erste Bohrung 22 angrenzt und zum offenen Ende hin sphärisch ausgebildet ist, wobei der Radius im wesentlichen gleich dem kugelsegmentförmigen Abschnitts des Kopfs 20 ist. Das Aufnahmeteil 21 weist ebenso wie das Aufnahmeteil 2 der ersten Ausführungsform eine zur Mitte des Teiles symmetrisch angeordnete U-förmige Ausnehmung 25 auf, deren Grund zur ersten Bohrung 22 hin gerichtet ist und durch die zwei freie Schenkel 26, 27 gebildet sind, deren freies Ende 28 den oberen Rand des Aufnahmeteils 21 bilden. In einem Bereich angrenzend an das freie Ende 28 weisen die Schenkel 26, 27 ein Innengewinde 29 auf. Das Innengewinde ist erfindungsgemäß als Flachgewinde, wie es bei der ersten Ausführungsform beschrieben wurde, ausgebildet.

Es ist ferner ein Druckelement 30 vorgesehen, das so ausgebildet ist, daß es an seiner dem Kopf 20 zugewandten Seite eine sphärische Ansenkung 31 aufweist, deren Radius im wesentlichen gleich dem Radius des kugelsegmentförmigen Abschnitts des Kopfes 20 ist. Der Außendurchmesser des Druckelements ist so gewählt, daß das Druckelement in dem Aufnahmeteil 21 eine Gleitbewegung durchführen kann, also zu dem Kopf 20 hin verschiebbar ist. Das Druckelement weist ferner eine koaxiale Bohrung 32 für den Zugriff auf eine Ausnehmung 33 in dem Schraubenkopf 20 zum Ineingriffbringen mit einem Eindrehwerkzeug auf.

Zum Fixieren des Stabes 100 und des Kopfes in seiner Winkelstellung ist ähnlich wie bei der ersten Ausführungsform eine Innenschraube 34 vorgesehen, die ein mit dem Innengewinde 29 der Schenkel zusammenwirkendes Außengewinde 35 aufweist. Das Außengewinde ist wiederum als Flachgewinde wie bei der ersten Ausführungsform ausgebildet.

Im Betrieb wird das Schraubenelement nach Einsetzen in das Aufnahmeteil 21 in den Knochen eingeschraubt. Dann werden nacheinander das Druckelement 30 und der Stab 100 eingesetzt. In diesem Stadium ist der Schraubenkopf 20 noch verschwenkbar. Durch Einschrauben der Innenschraube 34 werden das Schraubenelement und das Aufnahmeteil 21 zueinander und damit auch der Stab 100 fixiert. Da aufgrund der Ausbildung der zusammenwirkenden Gewinde der Schenkel und der Innenschraube als Flachgewinde keine Aufspreizung der Schenkel erfolgt, ist keine zusätzliche Sicherung erforderlich, wodurch die Polyaxialschraube kompakt gestaltet werden kann und kostengünstig hergestellt werden kann.

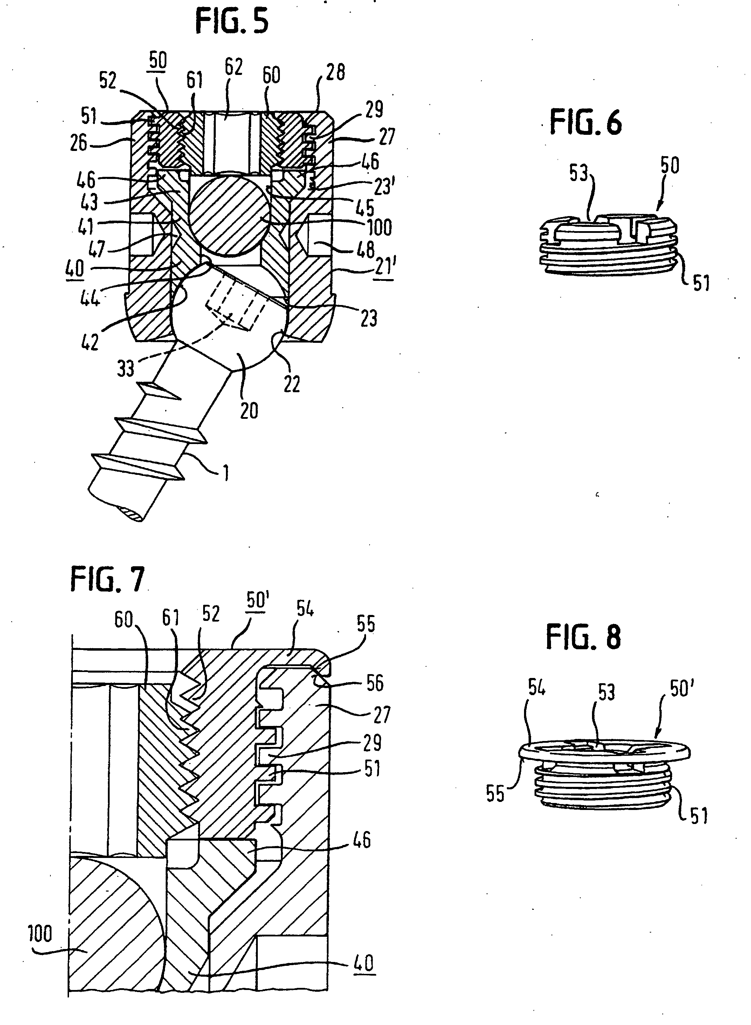

Die in Fig. 5 und 6 dargestellte dritte Ausführungsform zeigt ebenfalls eine Polyaxial-Knochenschraube. Der zweiten Ausführungsform entsprechende Teile sind mit denselben Bezugszeichen versehen. Die dritte Ausführungsform unterscheidet sich von der zweiten Ausführungsform durch die Ausbildung des Aufnahmeteils, des Druckelements und der Innenschraube.

Das Aufnahmeteil 21' weist angrenzend sein freies Ende 28 einen Abschnitt 23' auf der einen größeren Durchmesser aufweist, als die zweite Bohrung 23 und der sich konisch zu der ersten Bohrung hin verjüngt. Die Ausbildung des Aufnahmeteils bezüglich der U-förmigen Ausnehmung und der Schenkel 26, 27 ist wie bei der zweiten Ausführungsform. Angrenzend an das freie Ende 28 ist in einem vorbestimmten Bereich in dem Abschnitt 23' das Innengewinde 29 gebildet. Das Innengewinde ist wie bei der ersten und zweiten Ausführungsform als Flachgewinde ausgebildet.

Das Druckelement 40 dieser Ausführungsform weist einen im Wesentlichen zylindrischen ersten Abschnitt 41 aufweist, dessen Außendurchmesser so gewählt ist, dass das Druckelement in der zweiten Bohrung 23 des Aufnahmeteils 21' gleiten kann. In diesem ersten Abschnitt 41 ist eine sich zu dem Ende hin erweiternde kugelsegmentförmige Ausnehmung 42 vorgesehen, deren Kugelradius so gewählt ist, dass er in einem in das Aufnahmeteil eingesetzten Zustand den Kopf 2 des Schraubenelements teilweise umfasst. Am gegenüberliegenden Ende ist das Druckelement 40 in einem zweiten Abschnitt 43 mit einem gegenüber dem Durchmesser des ersten Abschnitts 41 vergrößerten Außendurchmesser gebildet, der größer ist als der Innendurchmesser der Bohrung 23 des Aufnahmeteils 21' aber kleiner als der Innendurchmesser zwischen den Schenkeln 26, 27. Der zweite Abschnitt 43 geht in den ersten Abschnitt 41 konisch entsprechend der Ausbildung des Aufnahmeteils über. Das Druckelement 40 weist ferner eine zentrale Bohrung 44 auf, die sich durch dieses hindurch erstreckt. Der Durchmesser der zentralen Bohrung 44 ist gerade so groß bemessen, dass ein Schraubwerkzeug zum Ineingriffbringen mit der in dem Kopf 20 vorgesehenen Ausnehmung 33 hindurchführbar ist.

Ein wesentlicher Unterschied zu dem Druckelement der zweiten Ausführungsform besteht darin , daß das Druckelement der dritten Ausführungsform in Richtung des freien Endes 28 der Schenkel verlängert ist. Dazu weist es an seinem der kugelsegmentförmigen Ausnehmung 42 gegenüberliegenden Ende eine U-förmige Ausnehmung 45 auf, wobei die Abmessungen der U-förmigen Ausnehmungen des Druckelements so bemessen sind, dass in den dadurch gebildeten Kanal der Stab 100 eingelegt werden kann. Die in Richtung der Zylinderachse des Aufnahmeteils 21' gesehene Tiefe der U-förmigen Ausnehmung 45 ist größer als der Durchmesser des aufzunehmenden Stabs 100, so dass das Druckelement 40 mit seitlichen Schenkeln 46 über den Stab 100 nach oben hervorsteht.

Das Druckelement 13 weist ferner auf seinem Mantel zwei um 90° versetzt zu der Mitte der U-förmigen Ausnehmung gegenüberliegend angeordnete und sich in radialer Richtung erstreckende Senkbohrungen 47 auf, die mit entsprechenden Kröpfbohrungen 48 im Mantel des Aufnahmeteils 21' zusammenwirken.

Anstelle der Innenschraube 9 der ersten und zweiten Ausführungsform ist bei der dritten Ausführungsform eine zwischen die Schenkel 26, 27 des Aufnahmeteils einschraubbare Mutter 50 mit einem Außengewinde 51 vorgesehen, welches mit dem Innengewinde 29 der Schenkel zusammenwirkt. Das Außengewinde 51 der Mutter ist als Flachgewinde, wie das der Innenschraube der vorherigen Ausführungsformen ausgebildet. Das Innengewinde 52 der Mutter 50 ist ein metrisches Gewinde. Wie aus Fig. 6 ersichtlich ist, weist die Mutter an ihrem einen Ende Schlitze 53 zum Ineingriffbringen mit einem Schraubwerkzeug auf.

Ferner ist eine Innenschraube bzw. Klemm- oder Setzschraube 60 zum Einschrauben in die Mutter 50 vorgesehen, welche ein metrisches Außengewinde 61 aufweist, das mit dem Innengewinde 52 der Mutter 50 zusammenwirkt. Die Innenschraube 60 weist eine Ausnehmung 62 zum Ineingriffbringen mit einem Schraubwerkzeug auf.

Im Betrieb wird wie bei der zweiten Ausführungsform zuerst das Schraubenelement in das Aufnahmeteil eingebracht. Anschließend wird, anders als bei der zweiten Ausführungsform das Druckelement 40 eingesetzt, welches über die Senk- und Kröpfbohrungen 47, 48 zunächst locker gehalten wird. Daran anschließend wird die Kochenschraube in den Knochen eingeschraubt und sodann der Stab 100 eingelegt. Daraufhin wird die Mutter 50 mit zunächst lose in diese eingeschraubter Innenschraube 60 eingeschraubt, bis die Mutter auf das obere Ende der Schenkel 46 stößt und damit das Druckelement 40 auf den Schraubenkopf 20 drückt, so daß dieser in dem Aufnahmeteil in seiner Stellung blockiert ist. Durch die Ausbildung des Innengewindes 29 der Schenkel und des Außengewindes der Mutter 50 als Flachgewinde erfährt die Mutter 50 keine radiale Kraftkomponente, sondern ausschließlich eine axial gerichtete Kraft, weshalb ein Aufspreizen der Schenkel nicht stattfindet. Somit ist der Schraubenkopf sicher blockiert. Weil die Schenkel 46 des Druckelements über den eingelegten Stab 100 hervorstehen, ist dieser noch verschiebbar, aber doch durch die Mutter 50 am Herausfallen bzw. Kippen gehindert. Zum Schluß wird die Innenschraube 60 festgezogen, bis sie auf den Stab 100 drückt und diesen fixiert. Die Fixierung des Stabes erfolgt somit unabhängig von der Fixierung des Kopfes.

Bei der in den Figuren 5 und 6 gezeigten dritten Ausführungsform ist die Mutter 50 und die Innenschraube 60 vollständig in das Aufnahmeteil 21'eingeschraubt. Bei der in den Figuren 7 und 8 gezeigten abgewandelten Ausführungsform weist die Mutter 50' an dem die Schlitze 53 beinhaltenden Ende einen ringförmigen Ansatz 54 auf, dessen Außendurchmesser dem Außendurchmesser des Aufnahmeteils 21' entspricht. Ferner ist das freie Ende des ringförmigen Ansatzes 54 kappenartig ausgebildet und weist eine schräge Fläche 55 auf, die in in das Aufnahmeteil 21' eingeschraubtem Zustand mit einer entsprechenden abgeschrägten Fläche 56 an dem freien Ende 28 des Aufnahmeteils 21' zusammenwirkt. Die Länge der Mutter 50' in axialer Richtung ist derart gewählt, dass in vollständig in das Aufnahmeteil 21' eingeschraubtem Zustand die Mutter 50' mit einer vorbestimmten Kraft auf das Druckelement 40 drückt, wobei der ringförmige Ansatz 54 als Anschlag zum Begrenzen dieser Kraft wirkt.

Der Betrieb erfolgt wie bei der dritten Ausführungsform mit dem einzigen Unterschied, daß die Mutter 50' nur bis zum Anliegen des ringförmigen Ansatzes 54 eingeschraubt werden kann und somit die auf das Druckelement wirkende Kraft auf einen vorbestimmten Wert eingestellt ist.

Zum Verhindern einer Verformung der Schenkel 26, 27 des Aufnahmeteils 21' durch axiale Verwringung verursacht durch Torsionskräfte im Moment des Festziehens greift der kappenartige Vorsprung 54 mit seiner abgeschrägten Fläche 55 auf die entsprechende abgeschrägte Fläche 56 an der Außenfläche des Aufnahmeteils. Nach dem endgültigen Festziehen ist eine derartige Torsionsbelastung nicht mehr vorhanden und die Schenkel 26, 27 erfahren keine nach außen wirkende Kraft, die ein Lockern der Mutter 50' hervorrufen könnte.

Alternativ zu der kappenartigen Ausbildung des Vorsprungs 54 kann ein an dem Einschraubwerk vorgesehener Ring verwendet werden.

In einer Abwandlung der oben beschriebenen Ausführungsformen ist anstelle des Gewindeschafts 1 ein Haken vorgesehen. In einer weiteren Abwandlung der polyaxialen Ausführungsformen ist anstelle des Gewindeschaftes 1 oder des Hakens eine Stange oder ein stabförmiges Element vorgesehen, das an beiden Enden einen kugelsegmentförmigen Kopf hat, der mit einem Aufnahmeteil der beschriebenen Art verbunden ist. Damit kann ein solches Element als Verbindungselement zwischen zwei Stäben 100 verwendet werden.

## Patentansprüche

1. Element mit einem Schaft (1) und einem damit verbundenen Halteelement (2; 21; 21') zum Verbinden mit einem Stab (100), wobei das Halteelement (2; 21; 21') eine einen U-förmigen Querschnitt aufweisende Ausnehmung zur Aufnahme des Stabes mit zwei an einem Ende (6; 28) freien Schenkeln (4,5; 26, 27) und einem Innengewinde (7; 29) an den freien Schenkeln und ein Verschlusselement (9; 34; 50; 50') mit einem Außengewinde (8; 35; 51), das mit dem Innengewinde der Schenkel zusammenwirkt, aufweist.
wobei die zwei Flanken (7a, 7b) des Innengewindes (7; 29) mit der Mittenachse (M) des Halteelements jeweils einen winkel von 90° einschließen und wobei
die Gewindequerschnitte im wesentlichen rechteckig ausgebildet sind.

2. Element nach Anspruch 1, **dadurch gekennzeichnet, dass** das mit dem Innengewinde (7; 23) zusammenwirkende Verschlusselement (9; 34; 50; 50') ein passendes Außengewinde (8; 35; 51) aufweist.

3. Element nach Anspruch 1 oder 2, **dadurch gekennzeichnet, dass** beim unbelasteten Ineingriffbringen von Verschlusselement (9; 34; 50; 50⁻) und freien Schenkeln (4,5; 25, 27) des Halteelements (2; 21; 21') ein Spalt zwischen den dem freien Ende abgewandten Flanken (7b) des Halteelements und den dem freien Ende zugewandten Flanken (8a) des Verschlusselements vorgesehen ist.

4. Element nach einem der Ansprüche 1 bis 3, **dadurch gekennzeichnet, dass** der Grund (7c) des Innengewindes der freien Schenkel jeweils abgerundet ausgebildet ist.

5. Element nach einem der Ansprüche 1 bis 4, **dadurch gekennzeichnet, daß** das Halteelement(2) mit dem Schaft (1) monoaxial verbunden ist.

6. Element nach einem der Ansprüche 1 bis 5, **dadurch gekennzeichnet, dass** das Verschlusselement (50; 50') als Mutter mit einem Innengewinde (52) ausgebildet ist und dass eine Innenschraube (60) zum Einschrauben in das Verschlusselement vorgesehen ist.

7. Element nach Anspruch 6, **dadurch gekennzeichnet, dass** das Innengewinde (52) des Verschlusselements (50, 50') und das Außengewinde (61) der Innenschraube (60) als metrische Gewinde ausgebildet sind.

8. Element nach einem der Ansprüche 1 bis 7, **dadurch gekennzeichnet, dass** das Element einen mit dem Schaft (1) verbundenen Kopf (20) aufweist, der mit dem Halteelement (21; 21') polyaxial verbunden ist und daß ein auf den Kopf (20) einwirkendes Druckelement (30; 40) zum Fixieren der Winkelstellung des Kopfes relativ zu dem Halteelement vorgesehen ist.

9. Element nach Anspruch 8, **dadurch gekennzeichnet, dass** das Druckelement (40) auf seiner dem Kopf (20) abgewandten Seite eine zu dem freien Ende (28) hin offene Ausnehmung (45) zur Aufnahme des Stabes (100) aufweist, deren Tiefe in axialler Richtung gesehen größer ist als der Durchmesser des Stabes (100).

10. Element nach einem der Ansprüche 1 bis 9, **dadurch gekennzeichnet, dass** das Verschlusselement (50; 50') an seinem einen Ende einen Vorsprung (54) in radialer Richtung aufweist, der als Anschlag beim Einschrauben des Verschlusselements zwischen die freien Schenkel (26, 27) dient.

11. Element nach Anspruch 10, **dadurch gekennzeichnet, dass** der Vorsprung (54) an seinem freien Ende einen Abschnitt (55) aufweist, der mit einem entsprechenden Abschnitt (56) an der Außenseite des Halteelements (21') zusammenwirkt und diesen kappenartig übergreift.

12. Element nach einem der Ansprüche 1 bis 11, **dadurch gekennzeichnet, dass** ein Abschnitt angrenzend an das freie Ende des Schaftes mit einem Knochengewinde zum Einschrauben in den Knochen oder als Haken ausgebildet ist.

13. Element nach einem der Ansprüche 1 bis 12, **dadurch gekennzeichnet, dass** die zwei Flanken (7a, 7b) des Innengewindes ohne Hinterschneidungen oder Stufen ausgebildet sind.

## Claims

1. Element with a shank (1) and a holding element (2; 21; 21') connected to it for connecting to a rod (100), wherein the holding element (2; 21; 21') comprises a recess having a U-shaped cross-section for receiving the rod with two legs (4, 5; 26, 27) open at one end (6; 28) and an inner thread (7; 29) on the open legs and a locking element (9; 34; 50; 50') with an outer thread (8; 35; 51) which cooperates with the inner thread of the legs, wherein the two flanks (7a, 7b) of the inner thread (7; 29) each enclose an angle of 90° with the central axis (M) of the holding element and wherein the thread cross-sections are substantially constructed as rectangular.

2. Element according to claim 1, **characterized in that** the locking element (9; 34; 50; 50') cooperating with the inner thread (7; 29) comprises a matching outer thread (8; 35; 51).

3. Element according to claim 1 or 2, **characterized in that** with unloaded bringing into engagement of the locking element (9; 34; 50; 50') and the open legs (4, 5; 26, 27) of the holding element (2; 21; 21') a gap is provided between the flanks (7b) of the holding element facing away from the open end and the flanks (8a) of the locking element facing the open end.

4. Element according to one of claims 1 to 3, **characterized in that** the root (7c) of the inner thread of the open legs is formed, respectively, as rounded.

5. Element according to one of claims 1 to 4, **characterized in that** the holding element (2) is monoaxially connected to the shank (1).

6. Element according to one of claims 1 to 5, **characterized in that** the locking element (50; 50') is constructed as a nut with an inner thread (52) and **in that** an inner screw (60) for screwing into the locking element is provided.

7. Element according to claim 6, **characterized in that** the inner thread (52) of the locking element (50; 50') and the outer thread (61) of the inner screw (60) are formed as metric threads.

8. Element according to one of claims 1 to 7, **characterized in that** the element has a head (20), which is connected to the shank (1) and polyaxially connected to the holding element (21; 21'), and **in that** a pressure element (30; 40) acting on the head (20) is provided for fixing the angle position of the head relative to the holding element.

9. Element according to claim 8, **characterized in that** the pressure element (40) on its side facing away from the head (20) comprises a recess (45) open to the open end (28) for receiving the rod (100), the depth of which, seen in the axial direction, is larger than the diameter of the rod (100).

10. Element according to one of claims 1 to 9, **characterized in that** the locking element (50; 50') on one of its ends comprises a projection (54) in the radial direction, which acts as a stop during screwing-in of the locking element between the open legs (26, 27).

11. Element according to claim 10, **characterized in that** the projection (54) comprises on its open end a section (55) which cooperates with a corresponding section (56) on the outer side of the holding element (21') and overlaps it cap-like.

12. Element according to one of claims 1 to 11, **characterized in that** a section adjacent to the open end of the shank is formed with a bone thread for screwing into the bone or as a hook.

13. Element according to one of claims 1 to 12, **characterized in that** the two flanks (7a, 7b) of the inner thread are constructed without undercuts or steps.

## Revendications

1. Elément avec une queue (1) et un élément de maintien (2; 21; 21') lui étant relié, pour assurer la liaison à une barre (100), l'élément de maintien (2; 21; 21') présentant un évidement, ayant une section transversale en forme de U, pour recevoir la barre, avec deux branches (4, 5; 26, 27) libres, à une extrémité (6; 28), et un filetage intérieur (7; 29) sur les branches libres, et un élément de fermeture (9; 34; 50; 50') muni d'un filetage extérieur (8; 35, 51), coopérant avec le filetage intérieur des branches, où
les deux flancs (7a, 7b) du filetage intérieur (7; 29) font, avec l'axe médian (M), chacun un angle de 90°, et où
les sections transversales de filetage sont sensiblement rectangulaires.

2. Elément selon la revendication 2, **caractérisé en ce que** l'élément de fermeture (9; 34; 50; 50'), coopérant avec le filetage intérieur (7; 29), présente un filetage extérieur (8; 35; 51) ajusté.

3. Elément selon la revendication 1 ou 2, **caractérisé en ce que**, lors de la mise en prise sans charge de l'élément de fermeture (9; 34; 50; 50') et des branches (4, 5; 26, 27) libres de l'élément de maintien (2; 21; 21'), un intervalle est prévu, entre les flancs (7b), opposés à l'extrémité libre, de l'élément de maintien et les flancs (8a), tournés vers l'extrémité libre, de l'élément de fermeture.

4. Elément selon l'une des revendications 1 à 3, **caractérisé en ce que** le fond (7b) du filetage intérieur des branches libres est chaque fois arrondi.

5. Elément selon l'une des revendications 1 à 4, **caractérisé en ce que** l'élément de maintien (2) est relié de façon mono-axiale à la queue (1).

6. Elément selon l'une des revendications 1 à 5, **caractérisé en ce que** l'élément de fermeture (50; 50') est réalisé sous forme d'écrou avec un filetage intérieur (52), et **en ce qu'**une vis intérieure (60) est prévue pour vissage dans l'élément de fermeture.

7. Elément selon la revendication 6, **caractérisé en ce que** le filetage intérieur (52) de l'élément de fermeture (50, 50') et le filetage extérieur (61) de la vis extérieure (60) sont réalisés sous forme de filetage métrique.

8. Elément selon l'une des revendications 1 à 7, **caractérisé en ce que** l'élément présente une tête (20), reliée à la queue (1) et reliée de façon poly-axiale à l'élément de maintien (21; 21') et **en ce qu'**un élément de pressage (30; 40), agissant sur la tête (20), est prévu pour fixer la position angulaire de la tête par rapport à l'élément de maintien.

9. Elément selon la revendication 8, **caractérisé en ce que** l'élément de pressage (40) présente, sur sa face opposée à la tête (20), un évidement (45) ouvert vers l'extrémité libre (28), afin de loger la barre (100), évidement dont la profondeur, en direction axiale, est supérieure au diamètre de la barre (100).

10. Elément selon l'une des revendications 1 à 9, **caractérisé en ce que** l'élément de fermeture (50; 50') présente, sur une de ses extrémités, une saillie (54), orientée en direction radiale et servant de butée lors du vissage de l'élément de fermeture entre les branches libres (26, 27).

11. Elément selon la revendication 10, **caractérisé en ce que** la saillie (54) présente, à son extrémité libre, un tronçon (55), coopérant avec un tronçon (56) correspondant, situé sur la face extérieure de l'élément de maintien (21') et entourant celui-ci à la façon d'un capuchon.

12. Elément selon l'une des revendications 1 à 11, **caractérisé en ce qu'**un tronçon est réalisé de façon limitrophe à l'extrémité libre de la queue, avec un filetage à os pour être vissé dans l'os, ou est réalisé en forme de crochet.

13. Elément selon l'une des revendications 1 à 12, **caractérisé en ce que** les flancs (7a, 7b) du filetage intérieur sont réalisés sans contre-dépouille ni étagement.
